# EUROPEAN PATENT APPLICATION

(11) **EP 2 062 585 A1**
(43) Date of publication of application: **27.05.2009**
(21) Application number: 07785340.6
(22) Date of filing: 14.08.2007
(51) Int. Cl.: A61K 31/7036, A61K 47/18, A61K 47/12, A61K 47/02, A61K 47/04, A61K 9/08, A61K 9/19, A61P 31/04, A61P 31/00

(54) **THE ANTIBIOTICS COMPOSITION COMPRISING AMINOGLYCOSIDE ANTIBIOTICS**

(30) Priority: 25.08.2006 CN 200610015439
(71) Applicant: Tianjin Hemey Bio-Tech Co., Ltd., Tianjin 300457 (CN)
(72) Inventor: ZHANG, Hesheng, Tianjin 300457 (CN)
(74) Representative: Hryszkiewicz, Danuta
(86) International application number: PCT/CN2007/002440
(87) International publication number: WO 2008/025227

(57) **Abstract**

The antibiotics composition comprising at least one of aminoglycoside antibiotics and at least one of ionic chelating agents used for inhibiting particulate formation, or at least one of buffers, or at least one of ionic chelating agents and buffers simultaneously. The composition can be formulated into solution, or combined with at least one of beta-lactam antibiotics, or combined with at least one of beta-lactam antibiotics and at least one of beta-lactamase inhibitors into solutions for controlling microbial infection in a container.

## Description

The invention relates to a pharmaceutical composition comprising: at least one aminoglycoside antibiotic; and (a) at least one ion-chelating agent, which inhibits the formation of aggregates in the composition; or (b) at least one buffer component; or (c) at least one ion-chelating agent and at least one buffer component. Optionally, the pharmaceutical composition further comprises at least one β-lactam antibiotic, or at least one β-lactam antibiotic and at least one β-lactamase inhibitor. The pharmaceutical composition of the invention is stable in liquid form and useful as an antimicrobial drug.

Aminoglycoside antibiotics are glycosides formed from aminosugars (monosaccharide or disaccharide) and aminocyclitols. They are alkaline in nature owing to their amino and other basic functional groups. The anti-bacterial mechanism of action of aminoglycoside antibiotics is that after entering bacteria the aminoglycoside antibiotic conjugates with the 30S subunit protein, which causes errors when tRNA translates mRNA code and results in non-functioning proteins inhibiting cell growth. Due to their broad anti-bacterial spectrum, high anti-bacterial activity, frequent clinical use, there have been more than 20 species of aminoglycosides developed since the discovery of the first aminoglycoside antibiotic, streptomycin, which was isolated from Streptothrix in 1940.

Another type of antibiotics widely used is β-lactam antibiotics. The anti-bacterial mechanism of action of β-lactam antibiotics is entirely different from that of aminoglycoside antibiotics. β-Lactam antibiotics inhibit the synthesis of bacterial cell wall by inhibiting the activity of D-alanyl-D-alanine transpeptidase (peptidoglycan transpeptidase) inside bacteria. Peptidoglycans are linear polysaccharide polypeptides with a network structure alternately comprising N-acetyl-glucosamine (Glc-NAc) and Mur-NAc. The transpeptide cross-linking reaction of these linear polymers catalysed by peptidoglycan transpeptidase results in network architecture and completes cell wall synthesis. β-Lactam antibiotics irreversibly inhibit the activity of the peptidoglycan transpeptidase and cause failure of bacterial cell wall formation. Without cell wall, bacterial cells don't have a definite shape and sustain high permeation pressure inside cells, which causes bacteriolysis resulting in the death of bacteria. β-Lactam antibiotics are the most widely used antibiotics in clinical medicine.

Bacteria have subsequently evolved to produce β-lactamase, which can hydrolyze the amido bond of the β-lactam ring of β-lactam antibiotics and transform β-lactam antibiotics into metabolites lacking antibacterial activity. In 1976, it was discovered that clavulanic acid separated from the fermented fluid of rod-like streptomycete was a unique β-lactamase inhibitor. Soon thereafter, other β-lactamase inhibitors, especially sulbactam and its lipid prodrugs, i.e., composition of ampicillin sodium and sulbactam sodium, and tazobactam became widely used in clinical settings.

It is generally known that the combination of β-lactam antibiotics with aminoglycoside antibiotics provides an anti-bacterial synergy. However, β-lactam antibiotics are acidic whereas aminoglycoside antibiotics are basic. When these two types of antibiotics are dissolved in the same solution, either a salt precipitates out due to acid-base reaction, or the amino group of the aminoglycoside antibiotic reacts with the β-lactam group of the β-lactam antibiotics. Both of the reactions drastically reduce the efficacy of these types of antibiotics. Therefore, mixing of these two types of antibiotics in the same solution is normally disadvantageous.

Further research has shown that when β-lactam antibiotics and aminoglycoside antibiotics are mixed together in one solution, aggregate particles are more likely to occur. In addition, the longer the solution stands, the more aggregate particles are generated. Aggregate particles in intravenous solution are harmful to the patient. Specifically, it has been found that infusion phlebitis is closely related to aggregate particle content in the infusion fluid (Remmington's Pharmaceutical Science, 18th Edition, Mark Publishing, 1990, p. 1567). This has become a clinical problem that needs to be solved. A pharmaceutical composition, in which β-lactam antibiotics and aminoglycoside antibiotics can be stabilized while maintaining efficacy would be particularly beneficial in clinical use.

Moreover, when a β-lactam antibiotic and an aminoglycoside antibiotic are combined in a mixture, the synergic bactericidal action can be achieved. Accordingly, providing a stable mixture of a β-lactam antibiotic and an aminoglycoside antibiotic would be of great social-economic significance.

The key in the development of a pharmaceutical composition comprising β-lactam antibiotics and aminoglycoside antibiotics which can be stabilized in solution with maintained efficacy is finding an agent which would promote solubility of β-lactam antibiotics and aminoglycoside antibiotics, yet also inhibit reaction between the β-lactam group of β-lactam antibiotics and the amino group of aminoglycoside antibiotics.

Particularly, it has been found that when the pH value is controlled in the range of between 3 and 9, a precipitate resulting from salt forming reaction and the reaction between the β-lactam group of β-lactam antibiotics with the amino group of aminoglycoside antibiotics can be inhibited to a certain extent. When the pH value is controlled in the range of between 4 and 8, the precipitate and the reaction between the amino group and β-lactam can be inhibited to a significant degree. And, when the pH value is controlled in the range of 6-7.5, the precipitate and the reaction between the amino group and β-lactam group can be completely inhibited. Strong ion chelating reagents can further inhibit the above-mentioned reactions between β-lactam antibiotics and aminoglycoside antibiotics.

In view of the above-described problems, it is one objective of the invention to provide a pharmaceutical composition which can be used as an anti-microbial and anti-infection drug.

In order to achieve the above objectives, in accordance with one embodiment of the invention, provided is a pharmaceutical composition, comprising: at least one aminoglycoside antibiotic, and (a) at least one ion-chelating agent, which inhibits the formation of aggregates in the composition; or (b) at least one buffer component; or (c) at least one ion-chelating agent and at least one buffer component. The pharmaceutical composition of the invention is stable in liquid form.

In a class of this embodiment, the composition is provided as a mixture with at least one β-lactam antibiotic in the same container. The resultant solution is clear and transparent without turbidity or precipitate, and the efficacy of the β-lactam antibiotic is maintained for at least 8 hours.

In another class of this embodiment, at least one β-lactam antibiotic and at least one β-lactamase inhibitor are simultaneously added to the pharmaceutical composition; the resultant solution is clear and transparent without turbidity or precipitate, and the efficacy of the aminoglycoside antibiotic, β-lactam antibiotic and β-lactamase inhibitor in the composition is maintained for at least 8 hours.

In another class of this embodiment, the aminoglycoside antibiotic is, without limitation, streptomycin, dibekacin, kanamycin, tobramycin, amikacin, arbekacin, gentamicin, sagamicin, isopamicin, sisomicin, netilmicin, neomycin, paromoycin, etimicin, astromicin, ribostamycin, micronomicin, spectinomycin, or a pharmaceutically acceptable salt or hydrate thereof. Particularly, the aminoglycoside antibiotic is amikacin, gentamicin, or etimicin.

In another class of this embodiment, the ion-chelating agent, which inhibits aggregate particle formation, is ethylenediamine tetraacetic acid (EDTA), diethylenetriaminepentaacetic acid (DTPA), hydroxyethylethylenediaminetriacetic acid (HEDTA), or a pharmaceutically acceptable salt thereof; particularly, the ion-chelating agent is EDTA, HEDTA, or a sodium salt thereof; and more particularly, the ion-chelating agent is EDTA disodium salt.

In another class of this embodiment, the buffer system is, without limitation, citric acid/citrate system or any other organic polyacid buffer system, phosphoric acid/phosphate system or any other inorganic acid buffer system, acetic acid/acetate system or any other organic monoacid system, arginine system or any other amino acid system, tris/HCl system, or any other pharmaceutically acceptable buffer system. Particularly, the buffer solution system is citric acid/citrate system, phosphoric acid/phosphate system, acetic acid/acetate system, arginine system, or carbonic acid/carbonate system. More particularly, the buffer system is citric acid/citrate system, phosphoric acid/phosphate system, or acetic acid/acetate system. The buffer component in the examples of this invention is sodium citrate.

In another class of this embodiment, the effective pH range of the buffer solution is between 4 and 8. Particularly, the effective pH range of the buffer solution is between 5.5 and 7.5. More particularly, the effective pH range of the buffer solution is between 6.0 and 6.75.

In another class of this embodiment, a concentration range of the buffer solution is between 1 and 500 mM; particularly, between 5 and 100 mM; and more particularly between 10 and 60 mM.

In another class of this embodiment, the β-lactam antibiotic is, without limitation, cefalothine, cefaloridine, cefazolin, cefapirin, cefaloglycin, cefalexin, cefadroxil, cefaclor, cefamandole, cefsulodine , cefoperazone, cefuroxime, cefotaxime, ceftizoxime, cefmenoxime, ceftriaxone, cefuzonam, cefixime, ceftazidime, ceftibuten, cefodizime, cephalosporin, cefpirome, cefepime, cefclidin, cefoxitin, cefmetazol, cefbuperazone, cefotetan, latamoxef, flomoxef, loracarbef, cefaloridine, latamoxef, cefminox, cefpiramide, cefonicid, ceforanide, cefacetrile, cefathiamidine, pheneticillin, propicillin, azidocillin, trityl penicillin, methicillin, nafcillin, oxacillin, cloxacillin, dicloxacillin, flucloxacillin, mecillinam, adicillin, ampicillin, amoxicillin, ticarcillin, carbenicillin, sulbenicillin, hetacillin, apalcillin, mezlocillin, temocillin, formidacillin, aspoxicillin, lenampicillin, azlocillin, piperacillin, pivampicillin, furbenicillin, phenoxymethypenicillin, apalcillin, nafcillin, metampicillin, or a pharmaceutically acceptable salt or hydrate thereof. Particularly, the β-lactam antibiotic is piperacillin, cefoperazone, ceftriaxone, cefodizime, or mezlocillin.

In another class of this embodiment, the β-lactamase inhibitor is, without limitation, clavulanic acid, sulbactam, sulbactam sodium, tazobactam, or a pharmaceutically acceptable salt or hydrate thereof. Particularly, the β-lactamase inhibitor is clavulanic acid, sulbactam, or tazobactam.

The clinical applications of the pharmaceutical compositions described herein include but are not limited to the following three classes:

1) In the first class is a pharmaceutical composition, comprising: at least one aminoglycoside antibiotic, a buffer solution, and an ion-chelating agent. This pharmaceutical composition, formulated as an injectable solution, is clear and transparent without turbidity or precipitate, and the efficacy of the β-lactam antibiotic and the aminoglycoside antibiotic in this composition is maintained for a prolonged period of time. A representative unit-dose composition in this class, comprises: between 0.01 g and 5 g of an aminoglycoside antibiotic, between 0.1 mg and 100 mg of EDTA, between 0.01 g and 5 g of sodium citrate. The pharmaceutical composition can be prepared as a solution preparation with freeze preservation, or as an injectable powder, or a lyophilized injectable powder, which is dissolved prior to use.

2) In the second class is a pharmaceutical composition, comprising: at least one aminoglycoside antibiotic and a buffer solution. This pharmaceutical composition, formulated as an injectable solution, is clear and transparent without turbidity or precipitate, and the efficacy of the β-lactam antibiotic and the aminoglycoside antibiotic in this composition is maintained for a prolonged period of time. A representative unit-dose composition in this class, comprises: between 0.01 g and 5 g of sodium citrate and between 0.01 g and 5 g of an aminoglycoside antibiotic. The pharmaceutical composition can be prepared as a solution preparation with freeze preservation, or as an injectable powder, or a lyophilized injectable powder, which is dissolved prior to use.

3) In the third class is a pharmaceutical composition, comprising: at least one aminoglycoside antibiotic and an ion-chelating agent. When the pharmaceutical composition, formulated as an injectable solution, is mixed in a container with at least a solution of a β-lactam antibiotic, a buffer component is required in order to obtain a clear and transparent mixture without turbidity or precipitate. The buffer component is particularly citric acid/citrate to adjust the pH value to between 6 and 7. The efficacy of the β-lactam antibiotic and the aminoglycoside antibiotic is maintained for a prolonged period of time. A representative unit-dose composition in this class, comprises: between 0.01 g and 5 g of an aminoglycoside antibiotic and between 0.1 mg and 100 mg of EDTA. The pharmaceutical composition can be prepared as a solution preparation stored with freeze preservation, or a lyophilized injectable powder, which is dissolved prior to use.

The pharmaceutical compositions described herein include, but are not limited, to the following representative unit dose formulations:

Formulation 1: gentamicin sulfate 10-2000 mg, and sodium citrate 10-5000 mg.

Formulation 2: streptomycin sulfate 10-2000 mg, and sodium citrate 10-5000 mg.

Formulation 3: dibekacin sulfate 10-2000 mg, and sodium citrate 10-5000 mg.

Formulation 4: kanamycin sulfate 10-2000 mg, and sodium citrate 10-5000 mg.

Formulation 5: tobramycin sulfate 10-2000 mg, and sodium citrate 10-5000 mg.

Formulation 6: amikacin sulfate10-2000 mg, and sodium citrate 10-5000 mg.

Formulation 7: arbekacin sulfate10-2000 mg, and sodium citrate 10-5000 mg.

Formulation 8: sagamicin sulfate10-2000 mg, and sodium citrate 10-5000 mg.

Formulation 9: isopamicin sulfate10-2000 mg, and sodium citrate 10-5000 mg.

Formulation 10: sisomicin sulfate10-2000 mg, and sodium citrate 10-5000 mg.

Formulation 11: netilmicin sulfate10-2000 mg, and sodium citrate 10-5000 mg.

Formulation 12: neomycin sulfate10-2000 mg, and sodium citrate 10-5000 mg.

Formulation 13: paromoycin sulfate10-2000 mg, and sodium citrate 10-5000 mg.

Formulation 14: etimicin sulfate10-2000 mg, and sodium citrate 10-5000 mg.

Formulation 15: gentamicin sulfate 10-2000 mg, disodium EDTA 0.1-100 mg, and sodium citrate 10-5000 mg.

Formulation 16: streptomycin sulfate 10-2000 mg, disodium EDTA 0.1-100 mg, and sodium citrate 10-5000 mg.

Formulation 17: dibekacin sulfate 10-2000 mg, disodium EDTA0.1-100 mg, and sodium citrate 10-5000 mg.

Formulation 18: kanamycin sulfate 10-2000 mg, disodium EDTA 0.1-100 mg, and sodium citrate 10-5000 mg.

Formulation 19: tobramycin sulfate 10-2000 mg, disodium EDTA 0.1-100 mg, and sodium citrate 10-5000 mg.

Formulation 20: amikacin sulfate 10-2000 mg, disodium EDTA 0.1-100 mg, and sodium citrate 10-5000 mg.

Formulation 21: arbekacin sulfate 10-2000 mg, disodium EDTA 0.1-100 mg, and sodium citrate 10-5000 mg.

Formulation 22: sagamicin sulfate 10-2000 mg, disodium EDTA0.1-100 mg, and sodium citrate 10-5000 mg.

Formulation 23: isopamicin sulfate 10-2000 mg, disodium EDTA0.1-100 mg, and sodium citrate 10-5000 mg.

Formulation 24: sisomicin sulfate 10-2000 mg, disodium EDTA 0.1-100 mg, and sodium citrate 10-5000 mg.

Formulation 25: netilmicin sulfate 10-2000 mg, disodium EDTA0.1-100 mg, and sodium citrate 10-5000 mg.

Formulation 26: neomycin sulfate 10-2000 mg, disodium EDTA 0.1-100 mg, and sodium citrate 10-5000 mg.

Formulation 27: paromoycin sulfate 10-2000 mg, disodium EDTA 0.1-100 mg, and sodium citrate 10-5000 mg.

Formulation 28: etimicin sulfate 10-2000 mg, disodium EDTA0.1-100 mg, and sodium citrate 10-5000 mg.

Formulation 29: gentamicin sulfate 10-2000 mg, and disodium EDTA 0.1-100 mg.

Formulation 30: streptomycin sulfate 10-2000 mg, and disodium EDTA 0.1-100 mg.

Formulation 31: dibekacin sulfate 10-2000 mg, and disodium EDTA 0.1-100 mg.

Formulation 32: kanamycin sulfate 10-2000 mg, and disodium EDTA 0.1-100 mg.

Formulation 33: tobramycin sulfate 10-2000 mg, and disodium EDTA 0.1-100 mg.

Formulation 34: amikacin sulfate 10-2000 mg, and disodium EDTA 0.1-100 mg.

Formulation 35: arbekacin sulfate 10-2000 mg, and disodium EDTA 0.1-100 mg.

Formulation 36: sagamicin sulfate 10-2000 mg, and disodium EDTA 0.1-100 mg.

Formulation 37: isopamicin sulfate 10-2000 mg, and disodium EDTA 0.1-100 mg.

Formulation 38: sisomicin sulfate 10-2000 mg, and disodium EDTA 0.1-100 mg.

Formulation 39: netilmicin sulfate 10-2000 mg, and disodium EDTA 0.1-100 mg.

Formulation 40: neomycin sulfate 10-2000 mg, and disodium EDTA 0.1-100 mg.

Formulation 41: paromoycin sulfate 10-2000 mg, and disodium EDTA 0.1-100 mg.

Formulation 42: etimicin sulfate 10-2000 mg, and disodium EDTA 0.1-100 mg.

An iso-osmotic solution may be used to prepare a parenteral solution of the pharmaceutical composition described in this invention. The iso-osmotic solution includes but is not limited to a glucose solution, a fructose solution, and normal saline. The unit dose range of glucose, fructose, or sodium chloride is between 0.1 g and 20 g; and the concentration range in the parenteral solution is between 0.1 % and 10% w/w.

Different formulations (vide supra) were prepared by selecting gentamicin, amikacin, or etimicin with one buffer component, or with one buffer component and disodium EDTA, one of five β-lactam antibiotics (piperacillin, cefoperazone, ceftriaxone, cefodizime, or mezlocillin) and one of two β-lactamase inhibitors (sulbactam or tazobactam).

Research on stability and intercomponent compatibility of three dosage forms and preservation methods was carried out. The results show that in solution preparations prepared by the following three ways: 1) used immediately after preparation; 2) prepared and cryopreserved, then thawed, and then added a β-lactam antibiotic prior to use; and 3) prepared as solution, freeze-dried, and preserved at low temperature, then reconstituted as a solution, and then added a β-lactam antibiotic prior to use; the contents of the β-lactam antibiotic, β-lactamase inhibitor and aminoglycoside antibiotic were all maintained at levels higher than 90%, and in some cases higher than 95%, which meets technical requirements for clinically applied multi-drug mixtures. In addition, the results show that EDTA can significantly reduce aggregate particle formation in the solution preparations of the compositions described in this invention. As a result, administration of the pharmaceutical composition of the invention by intravenous injection to treat microbial infection has become much safer.

The solution preparation of the pharmaceutical composition prepared for microbial control can be used as a parenteral solution, eye drops, nose drops, ear drops, genital duct drops, wash, or external use solution.

The solution preparation of the pharmaceutical composition may be prepared immediately prior to use; or it may be prepared as solution preparation, sealed, frozen preserved, and thawed at room temperature prior to use.

The pharmaceutical composition may be prepared as a solution, injectable powder, or freeze-dried injectable powder and preserved with cold-storage, and re-dissolved into a liquid solution with injectable fluid immediately prior to use.

Still in other aspects of the invention, provided is a method for the preparation of a solution, a freeze-dried injectable powder of the pharmaceutical composition in the present invention, comprising: (a) dissolving an aminoglycoside antibiotics, sodium citrate and/or disodium EDTA in injectable water, or in 2.5% injectable fructose aqueous solution, or in 5% normal saline; (b) adjusting the pH value to between 6 and 6.75; and sub-packaging the resultant solution in containers as unit-dose fluids; (c) placing the unit-dose fluids in a freeze-drier; adjusting the temperature of the freeze drier to minus 35°C, and pumping the atmosphere of the freeze drier to below 40 Pa; (d) adjusting the temperature to between 3 and 5°C, and removing water completely; (e) adjusting the temperature of the freeze drier to between 40 and 50°C and drying the obtained freeze-dried injectable powder; (f) sealing the bottles with sterile seal-capping; and (g) storing at below 5°C in the dark.

Table **1** lists partial HIAC test results. The results show that EDTA is effective in inhibiting formation of aggregate particles when the pharmaceutical composition is prepared in the form of a liquid solution.

The amounts of β-lactam antibiotics and β-lactamase inhibitor were determined using C18 reverse-phase LC and UV-VIS detector (Tianjin Hemay BioTech Co., Ltd., analysis method No. HM-K-03). The content of aminoglycoside antibiotics was measured using reverse-phase HPLC and evaporation-light-scattering detector (Tianjin Hemay BioTech Co., Ltd., analysis method No. HM-K-08). The amount of antibiotic component at each time point was expressed as a percentage with respect to the amount at the initial time (herein defined as 100%). The relative amount of each antibiotic component in the composition at each time point was expressed as peak area ratio with respect to the corresponding peak area of an external standard.

### Example 1

Pharmaceutical composition of cefoperazone sodium and gentamicin.

Cefoperazone sodium (4 g) was dissolved in 200 mL of injectable water, and gentamicin parenteral solution (80 mg in 2 mL of injectable water) was added. Upon mixing, a large amount of white solid precipitated out of the solution immediately.

### Example 2

Pharmaceutical composition of cefoperazone sodium, gentamicin, disodium EDTA, and buffer.

40 mg of cefoperazone sodium and 0.01 mg of disodium EDTA were dissolved in 2 mL of various buffer solutions having different pH values and concentrations. Then, 20 µL of gentamicin parenteral solution having a concentration of 40 mg/mL was added. The mixture was ultrasonicated for 5 minutes and clarity observed. Clear solutions were obtained, and there was no precipitate generated when the pH value of buffer solution was above 6. The type of buffer solution used had little effect on the results. The results are shown in the table below.

| **Buffer solution** | **pH of buffer solution** | **Concentration of buffer (mM)** | **Result** |
|---|---|---|---|
| Citric acid/sodium citrate | 5 | 10 | *** |
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| Acetic acid/sodium acetate | 6.5 | 20 | OK |
| Arginine/arginine sodium | 6.5 | 20 | OK |
| * the solution turned slightly turbid, **the solution turned turbid, ***the solution turned fairly turbid, OK: clear solution | | | |

### Example 3

Pharmaceutical composition of cefoperazone sodium, gentamicin, sulbactam sodium, disodium EDTA, and buffer.

40 mg of cefoperazone sodium, 0.01 mg of disodium EDTA, and 5 mg of sulbactam sodium were dissolved in 2 mL of various buffer solutions having different pH values and concentrations. Then, 20 µL of gentamicin parenteral solution having a concentration of 40 mg/mL was added. The mixture was ultrasonicated for 5 minutes and clarity observed. Clear solutions were obtained, and there was no precipitate generated when the pH value of buffer solution was above 6 and the concentration was higher than 20 mM. The type of buffer solution had little effect on the results. The results are shown in the table below.

| **Buffer solution** | **pH of buffer solution** | **Concentration of buffer solutions (mM)** | **Results** |
|---|---|---|---|
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| *the solution turned slightly turbid, **the solution turned turbid, ***the solution turned fairly turbid, OK: clear solution | | | |

### Example 4

Pharmaceutical composition of cefoperazone sodium, sulbactam sodium, gentamicin, and sodium citrate

0.20 g of sodium citrate was dissolved in 200 mL of injectable water, and the pH value was adjusted to 6.75 with citric acid. Then, gentamicin parenteral solution (160 mg/2 mL), cefoperazone sodium (4 g), and sulbactam sodium (0.5 g) were added, and the mixture was shaken.After 15 min of ultrasonication, a clear solution was obtained. The solution was packaged in a bottle or a bag and stored at room temperature. HIAC data at 1 hr and 20 hours were obtained (see Table **1**).

Observation of the appearance of the solution was conducted at 0, 1, 2, 4, and 6 hours. The amounts of cefoperazone sodium and sulbactam sodium were determined by sampling, and the results are shown below:

| **Time (h)** | **0** | **1** | **2** | **4** | **6** |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |

### Example 5

Pharmaceutical composition of cefoperazone sodium, sulbactam sodium, gentamicin, disodium EDTA, and sodium citrate

Sodium citrate (0.20 g) and disodium EDTA (1 mg) were dissolved in 200 mL of injectable water, the pH value was adjusted to 6.75 with citric acid, and gentamicin parenteral solution (160 mg in 2 mL) was added, and the mixture was shaken. Cefoperazone sodium (4 g) and sulbactam sodium (0.5 g) were added. After 15 min of ultrasonication, a clear solution was obtained. The solution was packaged in a bottle or a bag and stored at room temperature. HIAC data at 1 hr and 20 hours were determined (vide infra, Table **1**).

Observation of the appearance of the solution was conducted at 0, 1, 2, 4, and 6 hours. The amounts of cefoperazone sodium, sulbactam sodium, and gentamicin were determined by sampling, and the results are shown below:

| **Time (h)** | **0** | **1** | **2** | **4** | **6** |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |
| Gentamicin | 102.1 | 104.6 | 96.51 | 100.4 | NA |
| NA: undetermined content | | | | | |

### Example 6

Pharmaceutical composition of cefoperazone sodium, sulbactam sodium, etimicin sulfate, disodium EDTA, sodium citrate, and citric acid

Sodium citrate (0.20 g) and disodium EDTA (1 mg) were dissolved in 200 mL of injectable water, the pH value was adjusted to 6.5 with citric acid. Etimicin sulfate (200 mg) was added, and the mixture was shaken. Cefoperazone sodium (4 g) and sulbactam sodium (1.0 g) were added. After 15 min of ultrasonication, a clear solution was obtained. The solution was packaged in a bottle or a bag and stored at room temperature. HIAC data at 1 and 20 hours were determined (vide infra, Table **1**).

Observation of the appearance of the solution was conducted at 0, 1, 2, 4, 6 and 8 hours. The amounts of cefoperazone sodium, sulbactam sodium, and etimicin were determined by sampling, and the results are shown below:

| **Time (h)** | **0** | **1** | **2** | **4** | **6** | **8** |
|---|---|---|---|---|---|---|
| Cefoperazone sodium | 110.4 | 99.48 | 101.3 | 98.66 | 98.02 | 99.30 |
| Sulbactam sodium | 99.62 | 100.4 | 100.2 | 100.2 | NA | NA |
| Etimicin | 108.9 | 100.2 | 100.5 | 100.4 | 100.4 | 97.50 |
| NA: undetermined content | | | | | | |

### Example 7

Pharmaceutical composition of ceftriaxone sodium, sulbactam sodium, etimicin sulfate, disodium EDTA, sodium citrate and citric acid

Sodium citrate (0.20 g) and disodium EDTA (1 mg) were dissolved in 200 mL of injectable water, the pH value was adjusted to 6.5 with citric acid. Etimicin sulfate (200 mg) was added, and the mixture was shaken. Ceftriaxone sodium (4 g) and sulbactam sodium (1.0 g) were added. After 15 min of ultrasonication, a clear solution was obtained. The solution was packaged in a bottle or a bag and stored at room temperature.

Observation of the appearance of the solution was conducted at 0, 1, 2, 4, 6 and 8 hours. The amounts of ceftriaxone sodium, sulbactam sodium, and etimicin were determined by sampling, and the results are shown below:

| **Time (h)** | **0** | **1** | **2** | **4** | **6** | **8** |
|---|---|---|---|---|---|---|
| Ceftriaxone sodium | 97.01 | 100.3 | 96.40 | 99.01 | 98.91 | 99.53 |
| Sulbactam sodium | 96.84 | 94.43 | 93.35 | 93.23 | 92.13 | 93.38 |
| Etimicin | 100.7 | 101.0 | 101.0 | 101.0 | 100.8 | 100.7 |

### Example 8

Pharmaceutical composition of ceftriaxone sodium, tazobactam sodium, gentamicin sulfate, sodium citrate and citric acid

Sodium citrate (0.20 g) and enough citric acid were dissolved in 200 mL of injectable water to obtain a solution with the pH value 6.75. Gentamicin sulfate (160 mg/2 mL) was added, and the mixture was shaken. 20 mL of the resultant solution was collected. To those 20 mL, ceftriaxone sodium (20 mg) and tazobactam sodium (5 mg) were added, and small amount of white floc had precipitated out. After 15 min of ultrasonication, the white floc had dissolved to give a clear solution. The solution was packaged in a bottle or a bag and stored at room temperature.

Observation of the appearance of the solution was conducted at 0, 1, 2, 4, 6 and 8 hours. The amounts of ceftriaxone sodium and tazobactam sodium were determined by sampling, and the results are shown below:

| **Time (h)** | **0** | **1** | **2** | **4** | **6** | **8** |
|---|---|---|---|---|---|---|
| Ceftriaxone sodium | 98.75 | 100.1 | 99.97 | 98.87 | 99.27 | 100.5 |
| Tazobactam sodium | 102.2 | 97.53 | 94.16 | 94.58 | 95.45 | 94.44 |

### Example 9

Pharmaceutical composition of amikacin sulfate, mezlocillin sodium, disodium EDTA, sodium citrate and citric acid

Sodium citrate (0.20 g) and enough citric acid were dissolved in 200 mL of injectable water to give a solution with the pH value 6.5. Amikacin sulfate (500 mg) and disodium EDTA (1 mg) were added, shaken to give a clear solution.

100 mL of the resultant solution was filtered, then loaded into containers and placed in a freeze-drier. The temperature of the freeze drier was adjusted to minus 35°C, and the vacuum of the freeze drier was pumped to below 30 Pa. Then the temperature was adjusted to 3°C, and water was removed completely. The resultant freeze-dried injectable powder was dried at 40°C. The containers were sealed with sterile seal-caps, and stored in a refrigerator at 0°C. After 7 days, the freeze-dried injectable powder was dissolved in 100 mL of injectable water. 1 g of mezlocillin sodium added, shaken to give a clear solution.

Observation of the appearance of the solution was conducted at 0, 1, 2, 4, 6 and 8 hours. The amounts of mezlocillin sodium were determined by sampling at the above-mentioned time points, and the results are shown below:

| **Time (h)** | **0** | **1** | **2** | **4** | **6** | **8** |
|---|---|---|---|---|---|---|
| Mezlocillin sodium | 94.68 | 95.90 | 71.70 | 91.78 | 93.08 | 97.69 |

5 g of fructose was added to the remaining 100 mL of solution containing amikacin sulfate and disodium EDTA, and the mixture was shaken. A clear solution was obtained and stored in a refrigerator at minus 20°C. Seven days later, the solution was thawed and cefodizime sodium was added to 20 mL of the thawed solution. After 15 min of ultrasonication, a clear solution was obtained.

Observation of the appearance of the solution was conducted at 0, 1, 2, 4, 6 and 8 hours. The amounts of amikacin was determined by sampling at the above-mentioned time points, and the results are shown below:

| **Time (h)** | **0** | **1** | **2** | **4** | **6** | **8** |
|---|---|---|---|---|---|---|
| Amikacin | 96.88 | 97.78 | 96.14 | 96.88 | 98.34 | 98.11 |

### Example 10

Pharmaceutical composition of piperacillin sodium, sulbactam sodium, and etimicin sulfate.

4 g of piperacillin sodium, 1 g of sulbactam sodium were dissolved in 200 mL of injectable water. After filtration, 100 mL of the solution was packaged in a bottle or a bag, stored with cryopreservation (into solid). 7 days later, the solution was thawed at room temperature and 20 mL of the solution was collected, the pH value was adjusted to 6.0 with citric acid or sodium hydroxide, and 20 mg of freeze-dried powder of etimicin sulfate was added. After 10 min of ultrasonication, a clear solution was obtained, HIAC data at 1 and 20 hours were determined (see Table **1**).

Observation of the appearance of the solution was conducted at 0, 1, 2, 4, 6 and 8 hours. The amounts of piperacillin and sulbactam sodium were determined by sampling at the above-mentioned time points, and the results are shown below:

| **Time (h)** | **0** | **1** | **2** | **4** | **6** | **8** |
|---|---|---|---|---|---|---|
| Piperacillin | 101.8 | 101.3 | 101.8 | 101.2 | 98.3 | 95.1 |
| Sulbactam sodium | 94.1 | 97.4 | 96.4 | 97.7 | 96.9 | 97.1 |

### Example 11

Pharmaceutical composition of etimicin sulfate, disodium EDTA, piperacillin sodium, sulbactam sodium, and etimicin sulfate

100 mL of the solution obtained from Example 10 was filtered, then loaded into containers and placed in a freeze-drier. The temperature of the freeze drier was adjusted to minus 40°C, and the vacuum of the freeze drier was pumped to below 50 Pa. Then the temperature was adjusted to 3°C, and water was removed completely. The resultant freeze-dried injectable powder was dried at 45°C. The containers were sealed with sterile seal-caps, and stored in a refrigerator at 0°C. After 7 days, the freeze-dried injectable powder was dissolved in 100 mL of injectable water to give a clear solution. 60 mL of the solution was collected, the pH value was adjusted to 6.0 with citric acid or sodium hydroxide, and 20 mg of freeze-dried injectable powder of etimicin sulfate and 1 mg of disodium EDTA added. After 10 min of ultrasonication, a clear solution was obtained.

HIAC data at 1 hr and 20 hours were determined (see Table **1**).

Observation of the appearance of the solution was conducted at 0, 1, 2, 4, 6 and 8 hours. The amounts of piperacillin and etimicin were determined by sampling at the above-mentioned time points, and the results are shown below:

| **Time (h)** | **0** | **1** | **2** | **4** | **6** | **8** |
|---|---|---|---|---|---|---|
| Piperacillin | 101.8 | 101.3 | 101.8 | 101.2 | 98.3 | 95.1 |
| Etimicin | 94.81 | 99.66 | 100.9 | 101.5 | 96.97 | 94.44 |

### Example 12

Pharmaceutical composition of amikacin sulfate, piperacillin sodium, sulbactam sodium, and etimicin sulfate.

Citric acid or sodium hydroxide was added to 20 mL of the thawed solution from Example 10 to adjust the pH value to 6.0. 50 mg of amikacin sulfate was added. After 10 min of ultrasonication, a clear solution was obtained.

HIAC data at 1 hr and 20 hours were obtained (see Table **1**).

Observation of the appearance of the solution was conducted at 0, 1, 2, 4, 6 and 8 hours. The amounts of piperacillin and amikacin were determined by sampling at the above-mentioned time points, and the results are shown below:

| **Time (h)** | **0** | **1** | **2** | **4** | **6** | **8** |
|---|---|---|---|---|---|---|
| Piperacillin | 101.8 | 101.3 | 101.8 | 101.2 | 98.3 | 95.1 |
| Amikacin | 86.05 | 83.57 | 81.55 | 80.55 | 85.75 | 83.83 |

### Example 13

Pharmaceutical composition of amikacin sulfate, piperacillin sodium, sulbactam sodium, etimicin sulfate, and disodium EDTA.

Citric acid or sodium hydroxide was added to 20 mL of the thawed solution from Example 10 to adjust the pH value to 6.0. 50 mg of amikacin sulfate and 1 mg of disodium EDTA were added, after 10 min of ultrasonication, a clear solution was obtained.

HIAC data at 1 hr and 20 hours were obtained (see Table **1**).

### Example 14

Pharmaceutical composition of cefoperazone sodium, tazobactam sodium, amikacin sulfate, and disodium EDTA,

4 g of cefoperazone sodium, and 1.0 g of tazobactam sodium were dissolved in 100 mL of injectable water, and the pH value was adjusted to 6.75 with citric acid/sodium hydroxide. After 10 min of ultrasonication, a clear solution was obtained. 50 mL of the solution was collected, and 1 mg of disodium EDTA and 250 mg of amikacin sulfate were added. After 10 min of ultrasonication, a clear solution was obtained and stored at room temperature.

Observation of the appearance of the solution was conducted at 0, 1, 2, 4, 6 and 8 hours. The amounts of cefoperazone sodium and tazobactam sodium were determined by sampling at the above-mentioned time points, and the results are shown below:

| **Time (h)** | **0** | **1** | **2** | **4** | **6** | **8** |
|---|---|---|---|---|---|---|
| Cefoperazone sodium | 100.2 | 99.0 | 99.0 | 96.2 | 94.5 | 95.3 |
| Tazobactam sodium | 98.5 | 98.7 | 98.2 | 95.6 | 94.0 | 96.1 |

100 mL of the above-mentioned clear solution containing cefoperazone sodium and tazobactam sodium were separated, and 100 mg of etimicin sulfate was added. After 10 min of ultrasonication, a clear solution was obtained.

Observation of the appearance of the solution was conducted at 0, 1, 2, 4, 6 and 8 hours. The amounts of cefoperazone sodium and etimicin were determined by sampling at the above-mentioned time points, and the results are shown below:

| **Time (h)** | **0** | **1** | **2** | **4** | **6** | **8** |
|---|---|---|---|---|---|---|
| Cefoperazone sodium | 100.2 | 99.0 | 99.0 | 96.2 | 94.5 | 95.3 |
| Etimicin | 100 | 98.8 | 97.5 | 98.4 | 95.6 | 92.8 |

HIAC Test

Light obscuration testing was performed for solution preparations prepared with some of the compositions described in the examples according to the method described in the United States Pharmacopeia (USP 788) using HIAC-3000 light obscuration detector. Results are shown in Table **1**. Particle content is denoted by the number of particles per milliliter of solution. The value is an average of two measurements. Samples were tested at 1 hour and at 20 hours.

**Table 1: HIAC test results**

| **Sample** | **Aminoglycoside antibiotics** **(mg/mL)** | **Disodium EDTA** | **Number of particles (ppm)** | | | |
|---|---|---|---|---|---|---|
| | | | **1 hr** | | **20 hrs** | |
| | | **(mg/mL)** | **10 µm** | **25 µm** | **10** µ**m** | **25 µm** |
| Example 4 | G (0.8) | --- | 12 | 0 | 78 | 3 |
| Example 5 | G (0.8) | 0.005 | 17 | 0 | 24 | 0 |
| Example10 | Y (1.0) | --- | 58 | 0 | 169 | 6 |
| Example11 | Y (0.33) | 0.0167 | 29 | 2 | 42 | 4 |
| Example12 | A (2.5) | --- | 87 | 6 | 2862 | 23 |
| Example13 | A (2.5) | 0.05 | 49 | 3 | 371 | 7 |
| G: Gentamicin; Y: Etimicin; A: Amikacin | | | | | | |

This invention is not to be limited to the specific embodiments disclosed herein and modifications for various applications and other embodiments are intended to be included within the scope of the appended claims. While this invention has been described in connection with particular examples thereof, the true scope of the invention should not be so limited since other modifications will become apparent to the skilled practitioner upon a study of the drawings, specification, and following claims.

## Claims

1. A pharmaceutical composition comprising:
at least one aminoglycoside antibiotic or a pharmaceutically acceptable salt or hydrate thereof, and
(a) at least one ion-chelating agent, which inhibits the formation of aggregates in the composition;
(b) at least one buffer component; or
(c) at least one ion-chelating agent and at least one buffer component, and
a use of the pharmaceutical composition as an anti-microbial and anti-infection drug.

2. The pharmaceutical composition of claim 1, wherein said aminoglycosideantibiotic is etimicin, gentamicin, tobramycin, amikacin, netilmicin, dibekacin, kanamycin, arbekacin, sagamicin, isopamicin, sisomicin, neomycin, paromoycin, streptomycin, spectinomycin, micronomicin, astromicin, or ribostamycin, or a pharmaceutically acceptable salt or hydrate thereof.

3. The pharmaceutical composition of claim 1, wherein said ion-chelating agent is ethylenediamine tetraacetic acid (EDTA), diethylenetriaminepentaacetic acid (DTPA), hydroxyethylethylenediaminetriacetic acid (HEDTA), or a pharmaceutically acceptable salt or hydrate thereof.

4. The pharmaceutical composition of claim1, wherein said buffer component is citric acid/citrate, phosphoric acid/phosphate, acetic acid/acetate, arginine, carbonic acid/carbonate, or tris/HCl.

5. The pharmaceutical composition of claim 4, wherein an effective pH range of said buffer component is between 5.5 and 7.5.

6. The pharmaceutical composition of claim 4, wherein an effective pH range of said buffer component is between 6 and 6.75.

7. The pharmaceutical composition of claim 1, provided in a unit dose formulation, said unit dose comprising between 10 mg and 5 g of said aminoglycoside antibiotic.

8. The pharmaceutical composition of claim 1 or 3, provided in a unit dose formulation, said unit dose comprising between 0.1 mg and 100 mg of said ion-chelating agent.

9. The pharmaceutical composition of claim 1, provided as a solution preparation, injectable powder, or freeze-dried injectable powder.

10. A method for preparing the pharmaceutical composition provided as freeze-dried injectable powder of claim 9, the method comprising the steps of:
(a) dissolving each component of the pharmaceutical composition of claim 1 in injectable water, and adjusting the pH value with citrate sodium /citric acid to between 6 and 6.75;
(b) dividing solution obtained in step (a) into unit doses; placing each unit dose in an individual container; placing the containers in a freeze-drier at a temperature below minus 5°C; and adjusting the temperature of the freeze drier to about minus 35°C;
(c) evacuating the atmosphere of the freeze drier to below 40 Pa;
(d) adjusting the temperature in the freeze drier to between 3 and 5°C;
(e) removing water completely under the above-mentioned conditions to obtain freeze-dried injectable powder;
(f) adjusting the temperature of the freeze drier to between 40 and 50°C, and drying the freeze-dried injectable powder obtained in step (e); and
(g) charging nitrogen into the freeze drier, sealing the containers with sterile seal-capping, and storing the containers at a temperature below 5°C in the dark.
